(19) Europäisches Patentamt
European Patent Office
Office européen des brevets

(11) Veröffentlichungsnummer: **0 447 015 A1**

# (12) EUROPÄISCHE PATENTANMELDUNG

(21) Anmeldenummer: **91250078.2**

(51) Int. Cl.5: **A61K 47/48**, A61K 31/557

(22) Anmeldetag: **15.03.91**

(30) Priorität: **16.03.90 DE 4008925**

(43) Veröffentlichungstag der Anmeldung:
**18.09.91 Patentblatt 91/38**

(84) Benannte Vertragsstaaten:
**AT BE CH DE DK ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**W-1000 Berlin 65(DE)**

(72) Erfinder: **Buchmann, Bernd**
**Flemingstrasse 5a**
**W-1000 Berlin 21(DE)**
Erfinder: **Skuballa, Werner**
**Mattersburger Weg 12**
**W-1000 Berlin 28(DE)**
Erfinder: **Vorbrüggen, Helmut**
**Wilkestrasse 7**
**W-1000 Berlin 27(DE)**

## (54) 9-Halogenprostaglandin-Clatrathe und ihre Verwendung als Arzneimittel.

(57) Die Erfindung betrifft ß-Cyclodextrin-Clathrate von 9-Chlor-prostaglandinen der Formel I

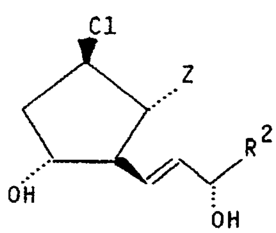

(I),

worin
Z die Reste

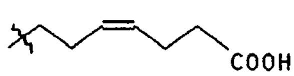 oder 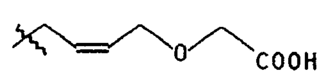

und $R^2$ die Reste 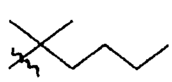 oder 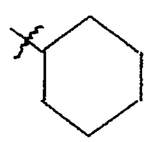

bedeuten.

EP 0 447 015 A1

Gegenstand der vorliegenden Erfindung sind ß- Cyclodextrin-Clathrate von 9-Chlor-prostaglandinanaloga und diese enthaltende Mittel.

9-Chlor- oder 9-Fluorprostaglandinanaloga sind pharmakologisch und medizinisch wertvolle Wirkstoffe, deren Herstellung und Anwendung in DE-OS 3724189, 3724190 und EP 299914 beschrieben sind. Diese Substanzen besitzen gegenüber den entsprechenden natürlichen Prostaglandinen bei ähnlichem Wirkungsspektrum eine wesentlich verbesserte Spezifität und vor allem wesentlich längere Wirkung.

In der europäischen Patentanmeldung EP 299 914 werden Cyclodextrinclathrate von 9-Halogenprostaglandinen der Formel

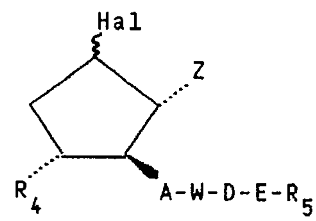

ganz allgemein beschrieben. Weder in den Beispielen noch sonst in der Beschreibung werden Cyclodextrine der genannten Prostaglandine im einzelnen genannt.

Es wurde nun gefunden, daß Einschlußverbindungen von 9-Chlor-$\Delta^4$ - bzw. 3-oxa-$\Delta^5$ -prostaglandinen mit ß-Cyclodextrin für galenische Zubereitungen besonders geeignet sind.

Die Erfindung betrifft Cyclodextrin-Clathrate von 9-Chlor-prostaglandinen der Formel I

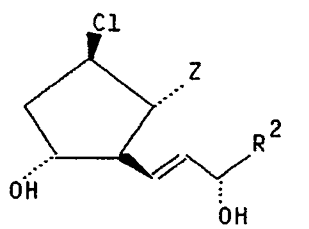

(I),

worin Z die Reste

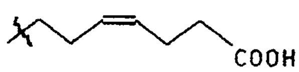 oder 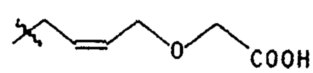

und $R^2$ die Reste 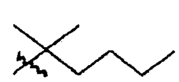 oder 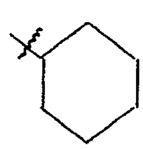

bedeuten.

Zur Herstellung der erfindungsgemäßen Clathrate werden die Verbindungen der allgemeinen Formel I entweder in einem pharmakologisch unbedenklichen Alkohol, vorzugsweise Ethanol, aufgelöst und zu wäßrigen Lösungen von ß-Cyclodextrin bei 60 °C zugesetzt, oder direkt in einer wäßrigen Suspension mit dem ß-Cyclodextrin mehrere Tage bei 29 °C gerührt. Nach der ersten Methodik kristallisieren die erhaltenen Clathrate beim Abkühlen aus. Diese Clathrate können durch Absaugen und Trocknen als feste, frei fließende Kristalle isoliert werden.

Die entsprechend dieser Erfindung hergestellten Clathrate sind wertvolle Pharmaka.

Die erfindungsmäßen Clathrate zeigen einen zytoprotektiven und ulkusheilenden Effekt, hemmen die Magensäuresekretion und wirken damit den unerwünschten Folgen nichtsteroidaler Entzündungshemmstoffe entgegen. Sie wirken außerdem an der Leber, Niere und an der Bauchspeicheldrüse zytoprotektiv.

Einige der erfindungsgemäßen Clathrate fördern die Nieren- und Hautdurchblutung, wobei diejenigen Clathrate die besonders die Hautdurchblutung fördern, beispielsweise zur Abheilung von Läsionen der Haut

eingesetzt werden können.

Einige der Clathrate wirken blutdrucksenkend, regulierend bei Herzrhythmusstörungen und hemmend auf die Plättchenaggregation mit den sich daraus ergebenden Einsatzmöglichkeiten, wie z.B. bei koronarer Herzkrankheit und Herzinfarkt. Die neuen Clathrate können auch in Kombination, z.B. mit ß-Blockern, Diuretika, Phosphodiesterasehemmern, Calciumantagonisten, Thromboxanantagonisten, Thromboxansynthetase- und Cyclooxygenasehemmern, gerinnungshemmenden Substanzen, wie auch Fibrinolytika, Leukotrienantagonisten, Leukotriensynthetasehemmern und Antigestagenen, verwendet werden.

Einige der erfindungsgemäßen Clathrate sind hervorragend geeignet zur Behandlung der allergischen und vasomotorischen Rhinitis, da sie eine rasche Abschwellung der Nasenschleimhaut bewirken.

Einige der erfindungsgemäßen Clathrate sind außerdem geeignet, nach enteraler oder parenteraler Applikation eine Menstruation zu induzieren oder eine Schwangerschaft zu unterbrechen.

Die erfindungsgemäßen Clathrate können in flüssigen oder festen galenischen Formulierungen verwendet werden, wobei die Formulierungen enteral, parenteral, vaginal oder rektal verabreicht werden können.

Zur Herstellung von Tabletten wird das Prostaglandin-ß-Cyclodextrin-Clathrat mit Trägersubstanzen und Hilfsstoffen wie Laktose, Maisstärke, Polyvinylpyrrolidon und Magnesiumstearat vermischt.

Zur Herstellung von Lösungen für die enterale und parenterale Anwendung werden die wäßrigen ß-Cyclodextrin-Clathrat-Lösungen zusammen mit Laktose lyophilisiert. Anschließend können die Lyophilisate mit physiologischer Kochsalzlösung auf die gewünschte Konzentration gebracht werden.

Die Erfindung umfaßt daher pharmazeutische Präparate und Formulierungen, die als Wirkstoff ein ß-Cyclodextrin-Clathrat eines 9-Chlor-prostaglandinanalogons enthalten.

Die Erfindung wird durch folgende Beispiele erläutert, ohne daß damit eine Begrenzung vorgenommen wird:

**BEISPIEL 1**

Zu einer Lösung von 2,14 g ß-Cyclodextrin in 14,5 ml Wasser tropft man bei 60 °C eine Lösung von 76 mg (4Z,13E)-(9R,11R,15R)-9-Chlor-11,15-dihydroxy-16,16-dimethyl-5,13-prostadiensäure in 1,0 ml Ethanol, rührt 2 Stunden bei 60 °C und läßt dann abkühlen. Nach 20 Stunden saugt man die Kristalle ab, wäscht mit 9,8 ml eines Gemisches Wasser/Ethanol im Verhältnis 1:1 nach und trocknet bei 40 °C 6 Stunden bei 0,1 Torr über Phosphorpentoxid. Man erhält 1,31 g frei fließende Kristalle des ß-Cyclodextrin-Clathrats des o.a. 9-Chlor-prostaglandin-Analogons.

Der Gehalt an 9-Chlor-prostaglandin-Analogon im Clathrat wird durch Säure-Base-Titration bestimmt und beträgt 5,0%.

**BEISPIEL 2**

Eine Mischung aus 21,6 g ß-Cyclodextrin und 900 mg (5Z,13E)-(9R,11R,15S)-9-Chlor-15-cyclohexyl-11, 15-dihydroxy-3-oxa-16, 17,18, 19,20-pentanor-5, 13-prostadiensäure in 186 ml Wasser wird für 6 Tage bei 22 °C gerührt. Man saugt die Kristalle ab und wäscht mit 15 ml eines Gemisches Wasser/Ethanol im Verhältnis 1:1 nach. Anschließend trocknet man 30 Stunden bei 22 °C bei 0,02 Torr über Phosphorpentoxid. Man erhält 17,1 g frei fließende Kristalle des ß-Cyclodextrin-Clathrats des o.a. 9-Chlor-prostaglandin-Analogons.

Der Gehalt an 9-Chlor-prostaglandin-Analogon im Clathrat wird durch Säure-Base-Titration bestimmt und beträgt 4,5%.

## Patentansprüche

**1.** ß-Cyclodextrin-Clathrate von 9-Chlor-prostaglandinen der Formel I

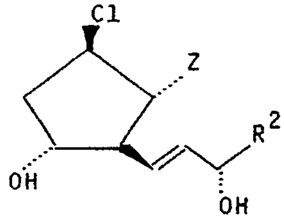

(I),

worin
Z die Reste

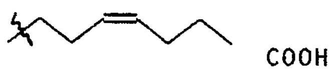 oder 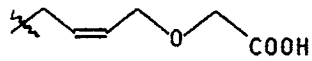

und $R^2$ die Reste 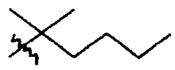 oder 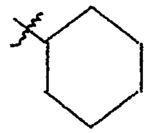

bedeuten.

2. Arzneimittel, enthaltend eine Verbindung aus Anspruch 1 und übliche Hilfs- und Trägerstoffe.

Europäisches Patentamt

# EUROPÄISCHER RECHERCHENBERICHT

Nummer der Anmeldung

**EP 91 25 0078**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X,Y | EP-A-0 299 914 (SCHERING A.G.)<br>* Seite 14, Beispiel 9; Patentansprüche *<br>– – – | 1,2 | A 61 K 47/48<br>A 61 K 31/557 |
| Y | WO-A-8 604 504 (SCHERING A.G.)<br>* Patentansprüche *<br>– – – – – | 1,2 | |
| | | | **RECHERCHIERTE SACHGEBIETE (Int. Cl.5)** |
| | | | A 61 K |

Der vorliegende Recherchenbericht wurde für alle Patentansprüche erstellt

| Recherchenort | Abschlußdatum der Recherche | Prüfer |
|---|---|---|
| Den Haag | 06 Juni 91 | SITCH W.D.C. |

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument